# EUROPEAN PATENT SPECIFICATION

(11) **EP 0 600 278 B1**
(45) Date of publication and mention of the grant of the patent: **21.07.1999**
(21) Application number: 93118298.4
(22) Date of filing: 11.11.1993
(51) Int. Cl.: C07K 14/705, C12N 15/09, G01N 33/68

(54) **Glutamate receptor protein and gene coding the same**
Protein und Gen des Glutamatrezeptors
Protéine et gène du récepteur de glutamate

(30) Priority: 13.11.1992 JP 30387892
(43) Date of publication of application: 08.06.1994
(73) Proprietor: Mitsubishi Chemical Corporation, Chiyoda-ku Tokyo (JP)
(72) Inventor: Mishina, Masayoshi, Niigata-shi, Niigata-ken (JP)
(74) Representative: TER MEER STEINMEISTER & PARTNER GbR

(56) References cited:
- WO-A-91/06648
- NATURE., vol.358, July 1992, LONDON GB pages 36 - 41 TATSUYA KUTSUWADA ET AL. 'Molecular diversity ...'
- NATURE., vol.357, May 1992, LONDON GB pages 70 - 74 HIROYUKI MEGURO ET AL. 'Functional characterization...'
- FEBS LETTERS, vol.313, no.1, 16 November 1992, AMSTERDAM NL pages 34 - 38 KAZUTAKA IKEDA ET A. 'Cloning and expression of the epsilon4...'

## Description

This invention relates to novel protein and gene coding the same, more specifically to protein NMDA (N-methyl-D-aspartic acid) type glutamate receptor which play a central role in nervous information transmission and gene (cDNAs) coding them.

It has been suggested that glutamate receptors are receptors of main stimulant nervous transmitter substances in a central nervous system of a higher animal, play a central role in nervous information transmission at a synapse, and also are deeply concerned with appearance of synapse plasticity which is basically required for memory and learning and neuronal cell death caused by a disease such as cerebral ischemia and epilepsy. Thus, it is considered that clarifications of molecular structures and functions of the glutamate receptors are required to understand a transmission mechanism of nervous information in a center, a cerebral structure of higher order and a disease of a brain. As in the case of receptors of acetylcholine and GABA (γ-aminobutyric acid), the glutamate receptors are roughly classified into ion channel type glutamate receptors and G protein coupled type receptors (metabolism-controlling type receptors). Receptors which can effect long-term reinforcement of synapse transmission at a CAl region of a hippocampus of which the most advanced study has been made about synapse plasticity are two kinds of ion channel type glutamate receptors. That is, an NMDA type receptor having Ca²⁺ permeability and opening depending on membrane potential and a quisqualate/kinate type receptor (or a non-NMDA type receptor). While the non-NMDA type receptor performs general synapse transmission, the NMDA type receptor performs Ca²⁺ permeation when a high frequent stimulus which induces long-term reinforcement of synapse transmission is given. The Ca²⁺ permeation is inhibited by Mg²⁺ depending on membrane potential. Although the glutamate receptors have important physiological functions as described above, molecular biological structures thereof had not been clarified for a long term. In recent years, by using a cDNA-producing system of Xenopus oocytes, Hollmann et al. cloned cDNA of a non-NMDA type glutamate receptor ("Nature", 342, pp. 643 to 648 (1989)) and Nakanishi et al. cloned cDNA of a rat-G protein coupled type glutamate receptor ("Nature", 349, pp. 760 to 765 (1991)) and cDNA of a rat-NMDA type receptor ("Nature", 354, pp. 31 to 37 (1991)). Other plural kinds of genes of glutamate receptors have been cloned, and the molecular biological mechanisms thereof have not yet been clarified sufficiently under the present situation.

The present inventors have paid special attention to NMDA type glutamate receptor and made researches and studied intensively, and consequently novel glutamate receptor have been obtained to accomplish the present invention.

That is, the characteristic features of the present invention reside in a glutamate receptor which has the amino acid sequence described in Sequence ID No. 1 of the Sequence Listing and a gene coding the same.

The drawings relating to the present invention are explained.

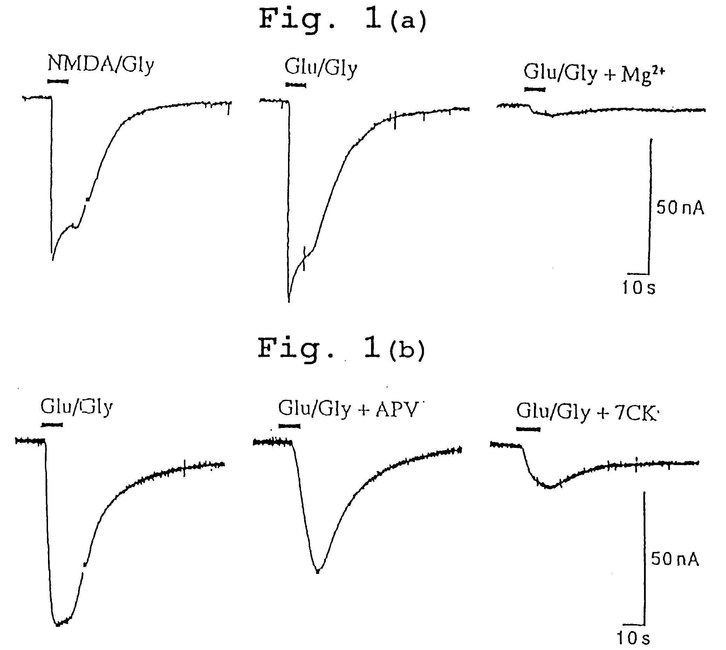

Fig. 1 shows functional expression of ε4/ζ1 heteromeric NMDA receptor channel cDNAs in frog oocytes. Current responses are measured at -70 mV membrane potential in normal Ringer's solution.
Fig. 1(a) represent a current response to 100 µM NMDA + 10 µM glycine (NMDA/Gly) of the ε4/ζ1 heteromeric channel, a current response to 10 µM L-glutamate + 10 µM glycine (Glu/Gly) of the same and 1 mM Mg²⁺ (Glu/Gly + Mg²⁺) on a current response to Glu/Gly of the same, respectively. Fig. 1(b) represent effects of 500 µM D-2-amino-5-phosphonovalerate (APV) (Glu/Gly + APV) and 100 M 7-chlorokynurenate (7CK) (Glu/Gly + 7CK) on a current response of the ε4/ζ1 heteromeric channel to Glu/Gly, respectively.

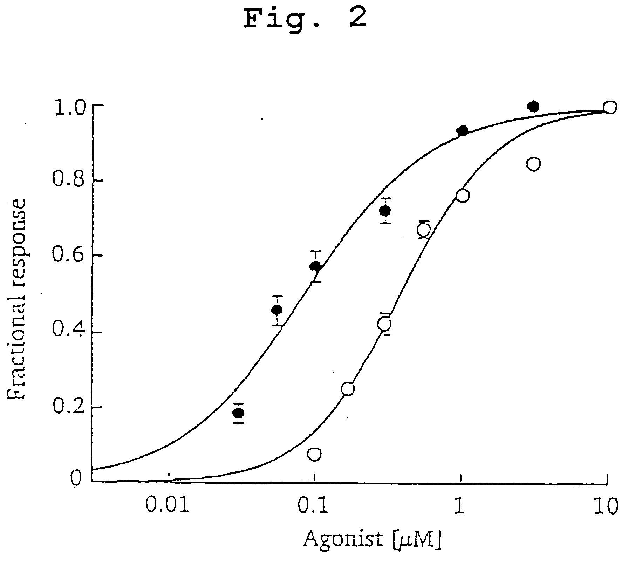

Fig. 2 shows pharmacological properties of the ε4/ζ1 heteromeric NMDA receptor channel. Current responses are measured at -70 mV membrane potential in a Ba²⁺-Ringer's solution. In the figure, dose-response relationship for L-glutamate (○) and glycine (●) in the presences of 10 µM glycine and 10 µM L-glutamate (each point represents the mean fractional responses obtained from 4 oocytes) are shown.

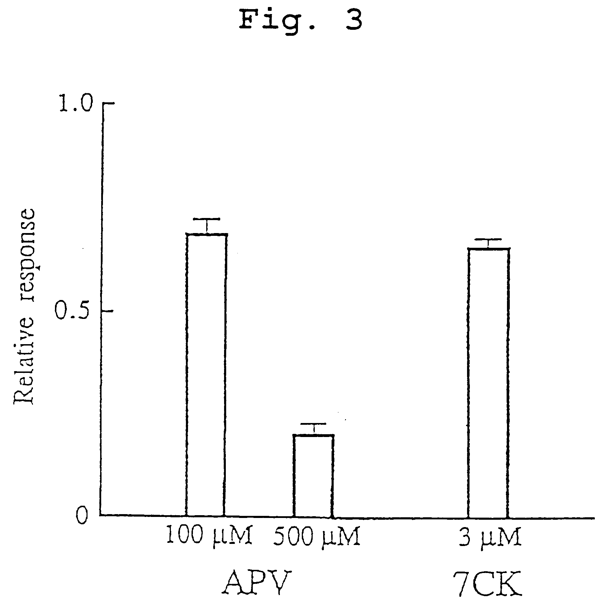

Fig. 3 shows pharmacological properties of the ε4/ζ1 heteromeric NMDA receptor channel. Current responses are measured at -70 mV membrane potential in a Ba²⁺-Ringer's solution. In the figure, influences of APV and 7CK on response to 4.7 µM L-glutamate + 0.9 µM glycine (the concentrations of agonists were 10-folds the respective EC₅₀ values) are shown.

In the following, the present invention is explained in detail. The glutamate receptor of the present invention has an amino acid sequence as shown in Sequence ID No. 1 of the Sequence Listing. The glutamate receptor shown in Sequence ID No.1 of the sequence table (hereinafter sometimes referred to as "ε4 subunit") is a protein comprising 1323 amino acids. This protein has a signal sequence, and a mature type thereof is considered to start from phenylalanine which is the 28th amino acid in Sequence ID No. 1 of the Sequence table. As a gene coding such a glutamate receptor, there may be mentioned, for example, a base sequence as shown in Sequence ID No. 1 of the Sequence Listing.

In the present invention, there may be included modified glutamate receptor in which amino acids or nucleic acids are partially removed, replaced or added within the range which does not impair activity as a glutamate receptor.

DNA fragments of the glutamate receptor of the present invention and the gene coding the same can be obtained by, for example, the following method.

First, cerebral tissues of a mammal such as mouse are homogenized in an aqueous solution containing guanidium thiocyanate or the like, and all RNAs are separated as precipitates by cesium chloride equilibrium density gradient centrifugation or sucrose density gradient centrifugation in accordance with the method of Chirgwin et al. ("Biochemistry", 18, pp. 5294 to 5299 (1979)). After separation, all RNAs are purified by extraction with phenol, precipitation with ethanol or the like, and the resulting RNAs are purified by oligo (dT) cellulose column chromatography to isolate poly (A)-containing mRNAs (poly A⁺ mRNAs) including mRNAs of the desired glutamate receptor, whereby an mRNA group can be obtained. The mRNA group prepared as described above and, for example, primer DNA as described in "FEBS Lett.", 272, pp. 73 to 80 (1990) are hybridized, and by using a reverse transcriptase and T4 DNA polymerase, cDNAs with double strands are synthesized and prepared according to a conventional method.

Then, EcoRI linkers are added to both ends of cDNA chains. The above cDNA chains are inserted into a position of EcoRI cleave site of a λ phage vector such as λgt10 to obtain a recombinant λ phage DNA group.

By using the recombinant λ phage DNA group obtained as described above and using an in vitro packaging kit such as commercially available Gigapack Gold (trade name, produced by Promega Co.) according to an operating manual, the so-called in vitro packaging was carried out to obtain λ phage particles having the recombinant λ phage DNAs. The λ phage particles obtained are increased by transforming host cells such as Escherichia coli according to a conventional method.

The clone group obtained is taken and collected on a nylon (trade name) film or a nitrocellulose film such as Gene Screening Plus (trade name, produced by Du Pont), and protein is removed in the presence of an alkali. The λ phage DNAs including cDNAs are hybridized with a ³²P-labeled probe prepared from fragments of cDNA of a mouse glutamate receptor which has been already cloned ("FEBS Lett.", 272, pp. 73 to 80 (1990)) and a DNA group of these cDNA clones, whereby clones having extremely high possibility of coding the desired glutamate receptor can be narrowed down to several clones.

For these clones, activity test is conducted by using translation of Xenopus oocytes to obtain cDNA clones coding the desired glutamate receptor.

The cDNA obtained as described above can be expressed in, for example, transient in vitro protein translation, specifically translation in Xenopus oocytes as described in "Nature", 329, pp. 836 to 838 (1987), or in a host such as a CHO cell transformed by a plasmid for expressing a protein prepared by connecting such a cDNA to downstream of a promoter of a expression plasmid for animal cells. Then, according to a conventional method, the expressed protein is collected to obtain the glutamate receptor of the present invention.

### EXAMPLES

The present invention is described in detail by referring to Examples, but the present invention is not limited by these Examples unless it is outside the scope thereof.

### Example 1

First, a cerebrum and a cerebellum of ICR mouse was homogenized in an aqueous solution of guanidium thiocyanate, and then extracted, separated and purified according to the method of Chirgwin et al. ("Biochemistry", 18, pp. 5294 to 5299 (1979)), and subjected to oligo (dT)-cellulose column chromatography according to the method of Aviv et al. ("Proc. Natl. Acad. Sci. USA", 69, pp. 1408 to 1412 (1972)) to isolate poly (A)-containing mRNAs (poly A⁺ mRNAs) containing mRNAs of an NMDA type glutamate receptor.

By using a cDNA synthesizing kit produced by Bethesda Research Laboratories Co., cDNAs having double strand were prepared from the poly A⁺ mRNAs. Both ends of the cDNAs were blunt-ended with T4 DNA polymerase, and the cDNAs were methylated by using an EcoRI-methylase. Then, EcoRI linkers were added to both ends of the cDNAs by ligase to prepare cDNA fragments having EcoRI restriction site at both ends by digestion with an EcoRI restriction enzyme.

The cDNAs obtained as described above were subjected to 1.5 % agarose gel electrophoresis to select and collect cDNAs having a size of 0.5 Kb or more.

The obtained cDNA fragments having a size of 0.5 Kb or more were linked to λgt10 phage DNAs by ligase, and the cDNAs were inserted into an EcoRI cleaved site of λgt10 to constitute a λgt10 cDNA library according to a conventional method. By using the λgt10 cDNA library obtained and using an in vitro packaging kit Gigapack Gold (trade name, produced by Promega Co.) according to an operating manual, in vitro packaging was carried out to obtain λ phage particles having recombinant λ phage DNAs. The λ phage particles obtained were cultivated by transforming Escherichia coli of host cells according to a conventional method.

Amino acid sequences highly preserved of mouse NMDA type receptor subunits, i.e. an oligo nucleotide sense primer, 5'-TGGAAT/CGGA/TATGATG/A/T/CGGG/A/T/CGA-3' (sequence ID No. 4 of the sequence table) corresponding to WNGMI/MGE (sequence ID No. 2 of the sequence table) existing at upstream end of the membrane spanning region M1 and an oligo nucleotide antisense primer, 5'-GCG/A/TGCT/CAG/AG/ATTG/A/TGCG/A/T/CG/ATG/ATA-3' (sequence ID No. 5 of the sequence table) corresponding to YTANLAA (sequence ID No. 3 of the sequence table) in M3 were synthesized. Polymerase chain reaction (PCR) was carried out using the double strand cDNA as a template and the above synthesized oligo nucleotides as primers.

PCR was carried out 30 cycles in total in which, after incubation at 94 °C for 3 minutes in 50 µl of the reaction solution containing 10 mM of Tris-HCl (pH 8.3), 50 mM of KCl, 1.5 mM of MgCl₂, 0.001 % of gelatin, 20 ng or less of a mouse cerebrum cDNA, 2 µM of the respective primers, 200 µM of 4 kinds of deoxynucleotide triphosphate and 4units of Tag polymerase; 94 °C for 1 minute; 50 °C for 1 minute;and 72 °C for 1.5 minutes were as one cycle.

After treating the PCR products by T4 DNA polymerase, they were inserted into the HincII site of pBluescript IISK (⁺) plasmid (Stratagene).

Subsequently, according to screening and base sequence determination, ε4 subunit cDNA clone was identified. The thus determined base sequence of the ε4 subunit cDNA and amino acid sequence expected therefrom were each shown in sequence ID No. 1 of the Sequence Listing.

A mouse cerebrum and cerebellum, cDNA library obtained from λgt10 was screened by using the ε4 subunit cDNA as a probe to obtain several clones which code the ε4 subunit. These phage-derived cDNA fragments were inserted into EcoRI site of the pBluescript IISK (-) plasmid (Stratagene) or pBKSA plasmid.

Base sequences of both chains of cDNA clones SE11 (from the -33rd to 2550th at the 5' end upstream in Sequence ID No. 1 of the sequence table) and SE4 (from the 2515th to +83st at the 3'end downstream in Sequence ID No. 1 of the Sequence Listing) were determined according to the dideoxy chain termination method using various primers synthesized by the DNA autosysnthesizer(available from Applied Biosystem Co.). Partial base sequences of respective clones, SE1 (the -33rd of the 5' upstream region to 2001st of the sequence No. ID No. 1 in the sequence table), TSEE6(the 1716th to 2291st of the sequence No. ID No. 1 in the Sequence Listing) and K1 (the 2013rd to +83rd of the 3' down stream region of the sequence No. ID No. 1 in the Sequence Listing) were completely identical with the sequences of the cDNA clones SE4 and SE11. The base sequences and the amino acid sequences were analyzed by GENETYX Software (SDC).

All coding area (the 1st to +3rd of the 3' down stream region in the Sequence ID No. 1 of the Sequence Listing, i.e. until stop codon) of the ε4 subunit cDNA was inserted by pSP35T plasmid and PCR method according to the PCR method between the NcoI and XbaI portions of the pSP35 plasmid (Cell, 66, pp. 257 to 270 (1991))to obtain pSPGR ε4 plasmid.

Then, by using the pSPGR ε4 plasmid cut by EcoRI as a template and using SP6 RNA polymerase, ε4-specific mRNAs were synthesized in vitro. Transcription was carried out in a solution containing ATP, TTP and CTP at a concentration of 0.5 mM, respectively and 0.1 mM GTP in the presence of 0.5 mM dinucleotide 7m GpppG having a cap structure(in which 2 Gs were linked at 5' and 7-site of one guanine was methylated, produced by P-L Biochemicals Co.).

The electrophysiological test was carried out by a conventional micropipet voltage clamp method. Oocytes generally have a membrane potential of about -30 to -40 mV. First, while a frog standard Ringer's solution (115 mM NaCl, 2.5 mM KCl, 1.8 mM CaCl₂ and 10 mM HEPES-NaOH (pH: 7.2)) was flown in a chamber, 2 glass microelectrodes charged with 3M KCl were stuck in the oocytes,and the membrane potential was fixed to -70 mV. In these oocytes, an agonist (NMDA) is linked to a glutamate receptor expressed on a cell membrane and an ion channel is opened, the oocytes have an action of returning membrane potential to an original value, whereby inflow and outflow of ions are caused. In the present experiment system, flow of these ions were detected, and opening and closing of the ion channel were observed.

The oocytes used were obtained from mature female Xenopus and separated into one oocyte in a Barth medium by using sharp tweezers and scissors. The concentration of the mRNAs was 0.1 µg/l and 10 µl thereof was injected into about 100 oocytes in an amount of 50 to 100 nl per one oocyte. The mRNAs were injected under a microscope by using a capillary and a micromanipulator. After the injection, the oocytes were incubated at 19 °C for one day in a Barth medium containing 0.1 mg/ml of gentamicin ("The Control of Gene Expression in Animal Development, Clarendon,Oxford (1974)). Then, the oocytes were allowed to stand in 1mg/ml of collagenase at room temperature for 1 hour, and then extracellular skins were removed under a microscope by using sharp tweezers. These oocytes were returned to the Barth medium containing 0.1 mg/ml of gentamicin, incubated again at 19 °C for one day and then used for an electrophysiological test.

The ε4 subunit-specific mRNAs (not more than 16 ng/egg) synthesized in vitro from a cloned cDNA and ζ1 NMDA type glutamate receptor subunit-specific mRNAs (not more than 13ng/egg) (FEBS Lett., 300, 39-45(1992)) described above were used singly or in combination, they were injected into the Xenopus oocytes.

In a frog standard Ringer's solution, the inward current of ε4 and ζ1 subunits expressing oocytes at -70 mV membrane potential was 70 ±9 nA (mean ±standard deviation, n = 13) with respect to 10 µM L-glutamate and 10 µM glycine and 68 ±13 nA (n = 8) with respect to 100 µM NMDA and 10 µM glycine (Fig. 1(a)). The current amplitude was extremely larger than that of the case where only the ζ1 NMDA type receptor subunit-specific mRNAs were injected to the oocytes (17 ±1 nA, n = 10, with respect to 10 µM L-glutamate and 10 µM glycine and 13 ±2 nA, n = 7, with respect to 100 µM NMDA and 10 µM glycine). The response of the heteromeric channel comprising the ε4/ζ1 subunit to 10 µM L-glutamate and 10 µM glycine was suppressed by 500 µM APV(D-2-amino -5-phosphonovalerate) which is a specific competitive antagonist of the NMDA receptors or 100µM of 7CK(7-chlorokynurenate) which had been reported as a competitive antagonist to glycine control site of the NMDA receptors (Fig. 2 and Fig. 3).

According to the above, it can be concluded that ε4 protein is a subunit of the NMDA receptor channels.

Since the suppressive effect of the NMDA receptor competitive antagonist to the ε4 subunit was relatively small, the pharmacological characteristics of the ε4/ζ1 heteromeric channel were quantitatively examined by using a Ba²⁺ Ringer's solution. The EC₅₀ values to L-glutamate or glycine obtained from the dose-reaction curve were 0.4 µM and 0.09 µM, respectively, and the Hill coefficients were 1.4 and 1.2, respectively (Fig. 2).

Apparent affinity of the ε4/ζ1 heteromeric NMDA receptor channel to an agonist was stronger than those of the ε1/ζ1, ε2/ζ1 and ε3/ζ1 heteromeric channels (Nature, 358, 36-41(1992)). The strengths in affinity to L-glutamate and glycine were in the order of ε4/ζ1 > ε3/ζ1 > ε2/ζ1. The effects of APV and 7CK were examined by 10-fold concentration of the EC₅₀ value (Fig. 3). Activities of the ε4/ζ1 channel were only decreased by 31 % in 100 µM APV and 34 % in 3 µM 7CK. The degree of inhibition was smaller than those of the other ε/ζ channels observed under the same conditions. The sensitivities to APV were ε1/ζ1 > ε2/ζ1 > ε3/ζ1 > ε4/ζ1, and those to 7CK were ε3/ζ1 > ε2/ζ1 > ε1/ζ1 = ε4/ζ1. According to the above, the ε4/ζ1 heteromeric channel was characterized that it has strong affinity to an agonist and weak sensitivity to a competitive antagonist.

The NMDA type glutamate receptor gene of the present invention are extected to be not only useful for clarifying nervous information transmission at a synapse, appearance of synapse plasticity which is basically required for memory and learning and neuronal cell death caused by a disease such as cerebral ischemia and epilepsy and understanding a transmission mechanism of nervous information in a center, a cerebral structure of higher order and a disease of a brain, but also useful for therapy of genetic diseases and preparation of novel pharmaceuticals (e.g. screening of an agonist or an antagonist).

(i) Applicant:                    MITSUBISHI KASEI CORPORATION
(A) Name:                         MITSUBISHI KASEI CORPORATION
(B) Street:                       5-2, Marunouchi 2-chome, Chiyoda-ku
(C) City:                         Tokyo
(D) State or Province:
(E) Country:                      Japan
(F) Postal Code:
(ii) Title of Invention: "Novel Proteins and Genes coding the same
(iii) Number of Sequences:    5
(iv) Computer-Readable Form:
(A) Medium Type                   Diskette 2DD
(B) Computer:                     PS/55
(C) Operating System:             MSDOS
(D) Software:                     TEXT FILE
(v) Current Application Data:
(A) Application Number:
(1) INFORMATION FOR SEQ ID NO:1
(i)SEQUENCE CHARACTERISTICS:
(A)LENGTH:3969 base pairs
(B)TYPE:nucleic acid
(C)STRANDEDNESS:double atranded
(D)TOPOLOGY:linear
(ii)MOLECULE TYPE:cDNA
(vi)ORIGINAL SOURCE
(A)ORGANISM:Mouse
(F)TISSUE TYPE:Brain
(xi)SEQUENCE DESCRIPTION:SEQ ID NO:1:
ATG CGC GGC GCC GGT GGC CCC CGC GGC CCT CGG GGC CCC GCT AAG ATG        48
Met Arg Gly Ala Gly Gly Pro Arg Gly Pro Arg Gly Pro Ala Lys Met

1               5                   10                  15

CTG TTG CTG CTG GCG CTG GCG TGC GCC AGC CCG TTC CCG GAG GAG GTG          96

Leu Leu Leu Leu Ala Leu Ala Cys Ala Ser Pro Phe Pro Glu Glu Val

                20                  25                  30

CCG GGG CCG GGC GCG GCC GGC GGG GGC ACG GGC GGG GCG CGG CCG CTC         144

Pro Gly Pro Gly Ala Ala Gly Gly Gly Thr Gly Gly Ala Arg Pro Leu

            35                  40                  45

AAC GTG GCG CTG GTC TTC TCT GGC CCG GCG TAC GCG GCC GAG GCG GCG         192

Asn Val Ala Leu Val Phe Ser Gly Pro Ala Tyr Ala Ala Glu Ala Ala

        50                  55                  60

CGC TTG GGC CCG GCC GTG GCG GCG GCA GTG CGC AGC CCG GGC CTG GAC         240

Arg Leu Gly Pro Ala Val Ala Ala Ala Val Arg Ser Pro Gly Leu Asp

    65                  70                  75                  80

GTA CGG CCC GTG GCG CTG GTG CTC AAC GGC TCC GAC CCT CGC AGC CTT         288

Val Arg Pro Val Ala Leu Val Leu Asn Gly Ser Asp Pro Arg Ser Leu

                    85                  90                  95

GTG CTG CAG CTC TGC GAC CTG CTG TCG GGG CTG CGC GTG CAC GGC GTG         336

Val Leu Gln Leu Cys Asp Leu Leu Ser Gly Leu Arg Val His Gly Val

                100                 105                 110

GTG TTC GAG GAC GAC TCG CGC GCG CCC GCC GTC GCG CCC ATT CTC GAC         384

Val Phe Glu Asp Asp Ser Arg Ala Pro Ala Val Ala Pro Ile Leu Asp

            115                 120                 125

TTC CTG TCG GCG CAG ACC TCG CTG CCC ATC GTG GCC GTG CAC GGC GGC         432

Phe Leu Ser Ala Gln Thr Ser Leu Pro Ile Val Ala Val His Gly Gly

        130                 135                 140

GCC GCG CTC GTA CTC ACA CCC AAG GAG AAG GGC TCC ACC TTC CTG CAG         480

Ala Ala Leu Val Leu Thr Pro Lys Glu Lys Gly Ser Thr Phe Leu Gln

145                 150                 155                 160

CTT GGC TCC TCC ACA GAG CAA CAG CTG CAG GTC ATT TTT GAG GTG CTG         528

Leu Gly Ser Ser Thr Glu Gln Gln Leu Gln Val Ile Phe Glu Val Leu
                165                 170                 175
GAG GAG TAC GAC TGG ACA TCC TTT GTG GCA GTG ACT ACG CGT GCC CCA 576
Glu Glu Tyr Asp Trp Thr Ser Phe Val Ala Val Thr Thr Arg Ala Pro
            180                 185                 190
GGC CAT CGA GCC TTC TTG TCA TAC ATC GAG GTG CTG ACT GAT GGC AGC 624
Gly His Arg Ala Phe Leu Ser Tyr Ile Glu Val Leu Thr Asp Gly Ser
        195                 200                 205
CTG GTG GGC TGG GAG CAT CGA GGA GCG CTG ACA CTG GAC CCC GGA GCG 672
Leu Val Gly Trp Glu His Arg Gly Ala Leu Thr Leu Asp Pro Gly Ala
    210                 215                 220
GGT GAG GCC GTC CTG GGC GCA CAG CTC CGT AGT GTC AGT GCG CAG ATC 720
Gly Glu Ala Val Leu Gly Ala Gln Leu Arg Ser Val Ser Ala Gln Ile
225                 230                 235                 240
CGC CTG CTC TTC TGC GCC CGC GAG GAG GCA GAG CCT GTT TTC CGG GCG 768
Arg Leu Leu Phe Cys Ala Arg Glu Glu Ala Glu Pro Val Phe Arg Ala
                245                 250                 255
GCA GAA GAG GCT GGT CTC ACT GGG CCT GGC TAC GTC TGG TTC ATG GTG 816
Ala Glu Glu Ala Gly Leu Thr Gly Pro Gly Tyr Val Trp Phe Met Val
            260                 265                 270
GGA CCT CAG CTG GCC GGA GGT GGG GGC TCC GGG GTC CCT GGG GAA CCA 864
Gly Pro Gln Leu Ala Gly Gly Gly Gly Ser Gly Val Pro Gly Glu Pro
        275                 280                 285
CTT CTT CTG CCA GGA GGT GCC CCA CTG CCT GCT GGG CTG TTT GCA GTG 912
Leu Leu Leu Pro Gly Gly Ala Pro Leu Pro Ala Gly Leu Phe Ala Val
    290                 295                 300
CGC TCT GCT GGC TGG CGT GAC GAC TTG GCA CGT CGA GTG GCT GCT GGT 960
Arg Ser Ala Gly Trp Arg Asp Asp Leu Ala Arg Arg Val Ala Ala Gly
305                 310                 315                 320

GTG GCG GTG GTG GCC AGA GGT GCC CAG GCC CTG CTG CGA GAC TAT GGC 1008
Val Ala Val Val Ala Arg Gly Ala Gln Ala Leu Leu Arg Asp Tyr Gly
                325                 330                 335
TTC CTG CCT GAG CTG GGC CAT GAC TGT CGC GCC CAG AAT CGC ACC CAC 1056
Phe Leu Pro Glu Leu Gly His Asp Cys Arg Ala Gln Asn Arg Thr His
            340                 345                 350
CGC GGG GAG AGT CTG CAC AGG TAT TTC ATG AAC ATC ACC TGG GAT AAC 1104
Arg Gly Glu Ser Leu His Arg Tyr Phe Met Asn Ile Thr Trp Asp Asn
        355                 360                 365
CGA GAC TAC TCC TTC AAT GAG GAT GGC TTT CTG GTA AAC CCG TCA CTG 1152
Arg Asp Tyr Ser Phe Asn Glu Asp Gly Phe Leu Val Asn Pro Ser Leu
    370                 375                 380
GTA GTC ATC TCC CTC ACC AGA GAC AGG ACG TGG GAA GTG GTG GGC AGC 1200
Val Val Ile Ser Leu Thr Arg Asp Arg Thr Trp Glu Val Val Gly Ser
385                 390                 395                 400
TGG GAA CAG CAG ACC CTC CGC CTC AAG TAC CCT CTA TGG TCC CGC TAT 1248
Trp Glu Gln Gln Thr Leu Arg Leu Lys Tyr Pro Leu Trp Ser Arg Tyr
                405                 410                 415
GGC CGC TTC CTG CAG CCG GTG GAT GAC ACG CAG CAC CTC ACT GTG GCC 1296
Gly Arg Phe Leu Gln Pro Val Asp Asp Thr Gln His Leu Thr Val Ala
            420                 425                 430
ACG CTG GAG GAG AGA CCT TTT GTC ATT GTA GAG CCT GCA GAC CCC ATC 1344
Thr Leu Glu Glu Arg Pro Phe Val Ile Val Glu Pro Ala Asp Pro Ile
        435                 440                 445
AGC GGC ACT TGC ATC AGA GAC TCG GTT CCC TGC CGG AGC CAG CTC AAC 1392
Ser Gly Thr Cys Ile Arg Asp Ser Val Pro Cys Arg Ser Gln Leu Asn
    450                 455                 460
CGT ACC CAC AGC CCT CCG CCT GAC GCT CCC CGC CCG GAG AAG AGA TGC 1440
Arg Thr His Ser Pro Pro Pro Asp Ala Pro Arg Pro Glu Lys Arg Cys

465                 470                 475                 480
TGC AAG GGT TTC TGC ATT GAC ATT TTG AAG AGG CTG GCG CAC ACC ATC 1488
Cys Lys Gly Phe Cys Ile Asp Ile Leu Lys Arg Leu Ala His Thr Ile
                485                 490                 495
GGT TTC AGC TAT GAC CTC TAC CTG GTT ACC AAC GGC AAG CAT GGC AAG 1536
Gly Phe Ser Tyr Asp Leu Tyr Leu Val Thr Asn Gly Lys His Gly Lys
            500                 505                 510
AAG ATC GAT GGC GTC TGG AAT GGC ATG ATT GGT GAG GTG TTC TAT CAG 1584
Lys Ile Asp Gly Val Trp Asn Gly Met Ile Gly Glu Val Phe Tyr Gln
        515                 520                 525
CGT GCG GAC ATG GCC ATC GGC TCC CTC ACC ATC AAT GAG GAG CGG TCA 1632
Arg Ala Asp Met Ala Ile Gly Ser Leu Thr Ile Asn Glu Glu Arg Ser
    530                 535                 540
GAG ATC GTG GAC TTC TCC GTC CCT TTT GTA GAG ACA GGC ATC AGC GTC 1680
Glu Ile Val Asp Phe Ser Val Pro Phe Val Glu Thr Gly Ile Ser Val
545                 550                 555                 560
ATG GTG GCA CGC AGC AAT GGC ACT GTG TCC CCC TCT GCC TTC CTC GAG 1728
Met Val Ala Arg Ser Asn Gly Thr Val Ser Pro Ser Ala Phe Leu Glu
                565                 570                 575
CCC TAC AGC CCC GCT GTG TGG GTG ATG ATG TTC GTC ATG TGC CTC ACC 1776
Pro Tyr Ser Pro Ala Val Trp Val Met Met Phe Val Met Cys Leu Thr
            580                 585                 590
GTG GTC GCC GTC ACA GTT TTC ATC TTT GAG TAC CTC AGT CCT GTG GGC 1824
Val Val Ala Val Thr Val Phe Ile Phe Glu Tyr Leu Ser Pro Val Gly
        595                 600                 605
TAT AAC CGA AGC CTG GCC ACG GGC AAA CGC CCC GGA GGC TCT ACC TTC 1872
Tyr Asn Arg Ser Leu Ala Thr Gly Lys Arg Pro Gly Gly Ser Thr Phe
    610                 615                 620
ACC ATT GGG AAA TCC ATC TGG CTG CTG TGG GCC CTG GTG TTC AAC AAC 1920

Thr Ile Gly Lys Ser Ile Trp Leu Leu Trp Ala Leu Val Phe Asn Asn
625                 630                 635                 640
TCC GTG CCA GTG GAG AAT CCT CGG GGC ACC ACC AGC AAG ATC ATG GTG     1968
Ser Val Pro Val Glu Asn Pro Arg Gly Thr Thr Ser Lys Ile Met Val
                645                 650                 655
CTG GTG TGG GCC TTC TTT GCC GTC ATC TTT CTT GCC AGC TAT ACA GCC     2016
Leu Val Trp Ala Phe Phe Ala Val Ile Phe Leu Ala Ser Tyr Thr Ala
            660                 665                 670
AAT CTG GCT GCC TTC ATG ATC CAG GAG GAG TAC GTG GAC ACC GTG TCT     2064
Asn Leu Ala Ala Phe Met Ile Gln Glu Glu Tyr Val Asp Thr Val Ser
        675                 680                 685
GGG CTC AGC GAC CGG AAG TTC CAG CGG CCC CAG GAG CAA TAC CCA CCC     2112
Gly Leu Ser Asp Arg Lys Phe Gln Arg Pro Gln Glu Gln Tyr Pro Pro
    690                 695                 700
CTG AAG TTT GGA ACG GTG CCC AAT GGG TCC ACG GAG AAG AAT ATC CGA     2160
Leu Lys Phe Gly Thr Val Pro Asn Gly Ser Thr Glu Lys Asn Ile Arg
705                 710                 715                 720
AGC AAC TAC CCT GAT ATG CAC AGC TAC ATG GTG CGA TAC AAC CAG CCA     2208
Ser Asn Tyr Pro Asp Met His Ser Tyr Met Val Arg Tyr Asn Gln Pro
                725                 730                 735
AGA GTG GAG GAG GCG CTC ACT CAG CTC AAG GCA GGG AAA CTG GAC GCC     2256
Arg Val Glu Glu Ala Leu Thr Gln Leu Lys Ala Gly Lys Leu Asp Ala
            740                 745                 750
TTC ATC TAT GAC GCA GCA GTG CTC AAC TAC ATG GCC CGA AAG GAT GAG     2304
Phe Ile Tyr Asp Ala Ala Val Leu Asn Tyr Met Ala Arg Lys Asp Glu
        755                 760                 765
GGC TGC AAG CTG GTC ACC ATC GGC TCA GGC AAG GTC TTC GCC ACC ACT     2352
Gly Cys Lys Leu Val Thr Ile Gly Ser Gly Lys Val Phe Ala Thr Thr
    770                 775                 780

GGT TAT GGC ATC GCC CTA CAC AAG GGC TCC CGC TGG AAG AGG CCC ATC 2400
Gly Tyr Gly Ile Ala Leu His Lys Gly Ser Arg Trp Lys Arg Pro Ile
785                 790                 795                 800
GAC CTG GCG CTG CTG CAG TTC CTG GGG GAC GAT GAG ATT GAG ATG CTG 2448
Asp Leu Ala Leu Leu Gln Phe Leu Gly Asp Asp Glu Ile Glu Met Leu
                805                 810                 815
GAG CGG CTG TGG CTT TCA GGG ATC TGC CAC AAC GAC AAA ATC GAG GTG 2496
Glu Arg Leu Trp Leu Ser Gly Ile Cys His Asn Asp Lys Ile Glu Val
            820                 825                 830
ATG AGC AGC AAG CTG GAT ATC GAC AAC ATG GCA GGT GTC TTC TAC ATG 2544
Met Ser Ser Lys Leu Asp Ile Asp Asn Met Ala Gly Val Phe Tyr Met
        835                 840                 845
CTC CTC GTG GCC ATG GGC CTC TCC TTG CTG GTC TTC GCC TGG GAA CAC 2592
Leu Leu Val Ala Met Gly Leu Ser Leu Leu Val Phe Ala Trp Glu His
    850                 855                 860
CTT GTG TAC TGG CGA CTG CGG CAC TGT CTG GGG CCC ACC CAC CGC ATG 2640
Leu Val Tyr Trp Arg Leu Arg His Cys Leu Gly Pro Thr His Arg Met
865                 870                 875                 880
GAT TTC CTA CTG GCC TTC TCC AGG GGT ATG TAC AGC TGC TGC AGC GCT 2688
Asp Phe Leu Leu Ala Phe Ser Arg Gly Met Tyr Ser Cys Cys Ser Ala
                885                 890                 895
GAG GCT GCT CCG CCG CCG GCC AAA CCC CCG CCA CCG CCG CAG CCG CTG 2736
Glu Ala Ala Pro Pro Pro Ala Lys Pro Pro Pro Pro Pro Gln Pro Leu
            900                 905                 910
CCC AGT CCG GCG TAT CCC GCC GCT CGC CCA CCC CCT GGC CCC GCA CCC 2784
Pro Ser Pro Ala Tyr Pro Ala Ala Arg Pro Pro Pro Gly Pro Ala Pro
        915                 920                 925
TTC GTG CCC CGA GAG CGC GCA GCC GCC GAC CGC TGG CGC CGG GCC AAG 2832
Phe Val Pro Arg Glu Arg Ala Ala Ala Asp Arg Trp Arg Arg Ala Lys

    930                 935                 940
GGC ACA GGG CCC CCG GGG GGC GCA GCG CTA GCC GAC GGC TTC CAC CGA 2880
Gly Thr Gly Pro Pro Gly Gly Ala Ala Leu Ala Asp Gly Phe His Arg
945                 950                 955                 960
TAC TAC GGC CCC ATC GAG CCG CAG GGG CTG GGC CTC GGC GAG GCG CGC 2928
Tyr Tyr Gly Pro Ile Glu Pro Gln Gly Leu Gly Leu Gly Glu Ala Arg
                965                 970                 975
GCG GCA CCG AGA GGC GCA GCC GGA CGC CCA CTG TCC CCA CCC ACC ACA 2976
Ala Ala Pro Arg Gly Ala Ala Gly Arg Pro Leu Ser Pro Pro Thr Thr
            980                 985                 990
CAG CCC CCA CAG AAG CCA CCA CCT TCC TAC TTC GCC ATC GTG CGC GAG 3024
Gln Pro Pro Gln Lys Pro Pro Pro Ser Tyr Phe Ala Ile Val Arg Glu
        995                 1000                1005
CAA GAG CCG GCC GAG CCC CCC GCC GGC GCC TTC CCG GGC TTT CCA TCT 3072
Gln Glu Pro Ala Glu Pro Pro Ala Gly Ala Phe Pro Gly Phe Pro Ser
    1010                1015                1020
CCG CCC GCT CCG CCT GCC GCC GCA GCC GCC GCC GTC GGG CCG CCA CTG 3120
Pro Pro Ala Pro Pro Ala Ala Ala Ala Ala Ala Val Gly Pro Pro Leu
1025                1030                1035                1040
TGC CGC CTG GCT TTC GAG GAC GAG AGC CCG CCC GCG CCC TCG GCT GGC 3168
Cys Arg Leu Ala Phe Glu Asp Glu Ser Pro Pro Ala Pro Ser Ala Gly
                1045                1050                1055
CGC GTT CTG ACC CCG AGA GCC AGC CGC TGT TGG GTG GGG GCG CGG GCG 3216
Arg Val Leu Thr Pro Arg Ala Ser Arg Cys Trp Val Gly Ala Arg Ala
            1060                1065                1070
GCC CGA GCG CTG GGG CCC CGA CCG CAC CAC CGC CGC GTC CGC ACC GCG 3264
Ala Arg Ala Leu Gly Pro Arg Pro His His Arg Arg Val Arg Thr Ala
        1075                1080                1085
CCA CCA CCG TGC GCC TAC CTG GAC CTC GAG CCT TCG CCT TCG GAC TCC 3312

Pro Pro Pro Cys Ala Tyr Leu Asp Leu Glu Pro Ser Pro Ser Asp Ser
    1090                1095                1100
GAG GAT TCG GAG AGC CTG GGC GGA GCG TCG CTC GGT GGC CTG GAG CCC 3360
Glu Asp Ser Glu Ser Leu Gly Gly Ala Ser Leu Gly Gly Leu Glu Pro
1105                1110                1115                1120
TGG TGG TTC GCC GAC TTC CCC TAC CCG TAT GCG GAG CGC CTC GGG CCG 3408
Trp Trp Phe Ala Asp Phe Pro Tyr Pro Tyr Ala Glu Arg Leu Gly Pro
                1125                1130                1135
CCG CCC GGC CGC TAC TGG TCG GTT GAC AAG CTC GGG GGC TGG CGC GCT 3456
Pro Pro Gly Arg Tyr Trp Ser Val Asp Lys Leu Gly Gly Trp Arg Ala
            1140                1145                1150
GGT AGC TGG GAC TAC CTG CCC CCG CGC GGC GGC CCC GCA TGG CAC TGC 3504
Gly Ser Trp Asp Tyr Leu Pro Pro Arg Gly Gly Pro Ala Trp His Cys
        1155                1160                1165
CGC CAC TGC GCC AGC CTG GAG CTG CTA CCG CCA CCA CGC CAT CTC AGC 3552
Arg His Cys Ala Ser Leu Glu Leu Leu Pro Pro Pro Arg His Leu Ser
    1170                1175                1180
TGC TCG CAC GAC GGC CTA GAC GGT GGC TGG TGG GCG CCT CCG CCT CCA 3600
Cys Ser His Asp Gly Leu Asp Gly Gly Trp Trp Ala Pro Pro Pro Pro
1185                1190                1195                1200
CCC TGG GCT GCG GGG CCA CCG GCC CCG CGC CGG GCG CGC TGT GGT TGT 3648
Pro Trp Ala Ala Gly Pro Pro Ala Pro Arg Arg Ala Arg Cys Gly Cys
                1205                1210                1215
CCG CGA CCG CAC CCG CAC CGC CCC CGG GCT TCG CAC CGT GCG CCC GCC 3696
Pro Arg Pro His Pro His Arg Pro Arg Ala Ser His Arg Ala Pro Ala
            1220                1225                1230
GCC GCA CCG CAC CAC CAC CGA CAC AGG CGC GCG GCG GGT GGC TGG GAC 3744
Ala Ala Pro His His His Arg His Arg Arg Ala Ala Gly Gly Trp Asp
        1235                1240                1245

CTC CCG CCG CCC GCG CCC ACC TCG CGT TCG CTG GAG GAC CTG AGC TCC 3792
Leu Pro Pro Pro Ala Pro Thr Ser Arg Ser Leu Glu Asp Leu Ser Ser
    1250                1255                1260
TGC CCA CGG GCG GCC CCC ACG CGC AGG CTC ACC GGG CCC TCG CGC CAC 3840
Cys Pro Arg Ala Ala Pro Thr Arg Arg Leu Thr Gly Pro Ser Arg His
1265                1270                1275                1280
GCG CGC CGC TGT CCG CAC GCT GCG CAT TGG GGG CCG CCC CTG CCC ACC 3888
Ala Arg Arg Cys Pro His Ala Ala His Trp Gly Pro Pro Leu Pro Thr
                1285                1290                1295
GCA TCT CAC CGG AGA CAC CGG GGC GGG GAC CTG GGC ACA CGC AGG GGC 3936
Ala Ser His Arg Arg His Arg Gly Gly Asp Leu Gly Thr Arg Arg Gly
            1300                1305                1310
TCT GCG CAC TTC TCC AGC CTG GAG TCC GAG GTA 3969
Ser Ala His Phe Ser Ser Leu Glu Ser Glu Val
        1315                1320
(1) INFORMATION FOR SEQ ID NO:2
(i)SEQUENCE CHARACTERISTICS:
(A)LENGTH:7 amino acids
(B)TYPE:amino acid
(D)TOPOLOGY:linear
(ii)MOLECULE TYPE:peptide
(vi)ORIGINAL SOURCE
(A)ORGANISM:Mouse
(F)TISSUE TYPE:Brain
(xi)SEQUENCE DESCRIPTION:SEQ ID NO:2:
Trp Asn Gly Met Xaa Gly Glu
1               5
Xaa : Ile or Met
(1) INFORMATION FOR SEQ ID NO:3

(i)SEQUENCE CHARACTERISTICS:
(A)LENGTH:7 amino acids
(B)TYPE:amino acid
(D)TOPOLOGY:linear
(ii)MOLECULE TYPE:peptide
(vi)ORIGINAL SOURCE
(A)ORGANISM:Mouse
(F)TISSUE TYPE:Brain
(xi)SEQUENCE DESCRIPTION:SEQ ID NO:3:
Tyr Thr Ala Asn Leu Ala Ala
1               5
(1) INFORMATION FOR SEQ ID NO:4
(i)SEQUENCE CHARACTERISTICS:
(A)LENGTH:20 base pairs
(B)TYPE:nucleic acid
(C)STRANDEDNESS:single stranded
(D)TOPOLOGY:linear
(ii)MOLECULE TYPE:other nucleic acid(anti-sense primer for PCR)
(iv)ANTI-SENSE:yes
(vi)ORIGINAL SOURCE
(A)ORGANISM:Mouse
(F)TISSUE TYPE:Brain
(xi)SEQUENCE DESCRIPTION:SEQ ID NO:4
TGG AAY GGW ATG ATN GGN GA 20
Y : T or C W : A or T N : A, T, G or C
(1) INFORMATION FOR SEQ ID NO:5
(i)SEQUENCE CHARACTERISTICS:
(A)LENGTH:20 base pairs
(B)TYPE:nucleic acid

(C)STRANDEDNESS:single stranded
(D)TOPOLOGY:linear
(ii)MOLECULE TYPE:other nucleic acid(anti-sense primer for PCR)
(iv)ANTI-SENSE:yes
(vi)ORIGINAL SOURCE
(A)ORGANISM:Mouse
(F)TISSUE TYPE:Brain
(xi)SEQUENCE DESCRIPTION:SEQ ID NO:5
GC DGC YAR RTT DGC NRT RTA 20
D : G, A or T Y : T or C R : A or G
N : A, T, G or C

## Claims

1. A glutamate receptor which has the amino acid sequence described in Sequence ID No. 1 of the Sequence Listing.

2. The glutamate receptor according to Claim 1, wherein the receptor has an amino acid sequence starting from the 28th amino acid in Sequence ID No. 1 of the Sequence Listing.

3. A gene coding the glutamate receptor according to Claim 1 or 2.

4. The gene according to Claim 3 which has the base sequence described in Sequence ID No. 1 of the Sequence Listing.

5. A method for effecting screening of agonist or antagonist which binds to a glutamate receptor which comprises using the glutamate receptor according to Claim 1.

## Patentansprüche

1. Glutamatrezeptor, welcher die in Sequenz ID Nr. 1 der Sequenzliste beschriebene Aminosäuresequenz aufweist.

2. Glutamatrezeptor nach Anspruch 1, wobei der Rezeptor eine Aminosäuresequenz aufweist, welche mit der 28sten Aminosäure in Sequenz ID Nr. 1 der Sequenzliste beginnt.

3. Den Glutamatrezeptor nach Anspruch 1 oder 2 kodierendes Gen.

4. Gen nach Anspruch 3, welches die in Sequenz ID Nr. 1 der Sequenzliste beschriebene Basensequenz aufweist.

5. Verfahren zur Bewirkung eines Screenings von Agonist oder Antagonist, welcher an einen Glutamatrezeptor bindet, umfassend die Verwendung des Glutamatrezeptors nach Anspruch 1.

## Revendications

1. Récepteur du glutamate possédant la séquence d'acides aminés décrite dans la séquence n°1 de la liste de séquences.

2. Récepteur du glutamate selon la revendication 1, ledit récepteur possédant une séquence d'acides aminés démarrant au niveau du 28ème acide aminé de la séquence n°1 de la liste de séquences.

3. Gène codant pour le récepteur du glutamate de la revendication 1 ou 2.

4. Gène selon la revendication 3, qui possède la séquence de bases décrite dans la séquence n°1 de la liste de séquences.

5. Procédé destiné à effectuer le criblage d'un agoniste ou d'un antagoniste qui se lie au récepteur du glutamate, comprenant l'utilisation du récepteur du glutamate selon la revendication 1.
